# FASCICULE DE BREVET EUROPEEN

(11) **EP 2 147 089 B1**
(45) Date de publication et mention de la délivrance du brevet: **22.01.2014**
(21) Numéro de dépôt: 08787835.1
(22) Date de dépôt: 21.03.2008
(51) Int. Cl.: C11C 3/04, B01J 27/16, C07C 67/03, B01J 21/06

(54) **PROCEDE DE FABRICATION D'ESTERS ALCOOLIQUES A PARTIR DE TRIGLYCERIDES ET D'ALCOOLS AU MOYEN DE CATALYSEURS HETEROGENES A BASE DE PHOSPHATE OU DE COMPOSE ORGANOPHOSPHORE DE ZIRCONIUM**
VERFAHREN ZUR HERSTELLUNG ALKOHOLISCHER ESTER AUS TRIGLYCERIDEN UND ALKOHOLEN UNTER VERWENDUNG HETEROGENER KATALYSATOREN MIT EINEM PHOSPHAT ODER EINER ORGANOPHOSPHORIERTEN VERBINDUNG VON ZIRCONIUM
METHOD FOR PRODUCING ALCOHOLIC ESTERS FROM TRIGLYCERIDES AND ALCOHOLS USING HETEROGENEOUS CATALYSTS CONTAINING PHOSPHATE OR AN ORGANOPHOSPHORATED COMPOUND OF ZIRCONIUM

(30) Priorité: 12.04.2007 FR 0702675
(43) Date de publication de la demande: 27.01.2010
(73) Titulaire: IFP Energies nouvelles, 92852 Rueil-Malmaison Cedex (FR)
(72) Inventeur: LECOCQ, Vincent, F-69530 Brignais (FR); MAURY, Sylvie, F-69390 Charly (FR); BAZER-BACHI, Delphine, F-69230 Saint Genis Laval (FR)
(86) Numéro de dépôt international: PCT/FR2008/000389
(87) Numéro de publication internationale: WO 2008/135665

(56) Documents cités:
- WO-A-2005/021697
- FR-A- 2 698 101
- US-A- 3 416 884
- US-A1- 2004 007 532
- DE FILIPPIS P ET AL: "Rapeseed Oil Transesterification Catalyzed by Sodium Phosphates" ENERGY & FUELS, THE SOCIETY, WASHINGTON, DC, US, vol. 19, no. 6, 2005, pages 2225-2228, XP003010535 ISSN: 0887-0624 cité dans la demande
- DEMIRBAS ET AL: "Biodiesel production from vegetable oils via catalytic and non-catalytic supercritical methanol transesterification methods" PROGRESS IN ENERGY AND COMBUSTION SCIENCE, ELSEVIER SCIENCE PUBLISHERS, AMSTERDAM, NL, vol. 31, no. 5-6, 2005, pages 466-487, XP005213717 ISSN: 0360-1285
- KAMIYA Y; SAKATA S; YOSHINAGA Y; OHNISHI R; OKUHARA T: "Zirconium phosphate with a high surface area as a water-tolerant solid acid" CATALYSIS LETTERS, vol. 94, no. 1-2, avril 2004 (2004-04), pages 45-47, XP002456899

## Description

La présente invention est relative à un nouveau procédé de fabrication d'esters alcooliques d'acides monocarboxyliques à partir de corps gras d'origine végétale ou animale.

La réaction principalement visée est une transestérification réalisée selon le schéma I ci-dessous et éventuellement une réaction couplée estérification et transestérification, l'estérification étant réalisée selon le schéma II ci-dessous.

Schéma I : 1 triglycéride + 3 alcools → 3 esters de corps gras + glycérine

Schéma II : Acide gras + alcool → esters d'acide gras + eau

Acide gras + glycérine → glycéride + eau

Les esters de corps gras sont actuellement utilisés dans de nombreuses applications comme carburants diesel, fuels domestiques, solvants écologiques, composés de base pour la fabrication de sulfonates d'alcools gras, d'amides, de dimères d'esters, etc.

Dans le cas du carburant Diesel, qui constitue aujourd'hui une application majeure des esters de corps gras, un certain nombre de spécifications ont été établies dont la liste, les limites et les méthodes font partie de la norme EN 14214 (2003) applicable actuellement en Europe. L'ester doit contenir au moins 96,5 % en masse d'esters, au plus 0,8 % en masse de monoglycérides, au plus 0,2 % en masse de diglycérides et au plus 0,2 % en masse de triglycérides, peu d'acides gras libres (<0,5 mg de KOH par g), qui peuvent être corrosifs, moins de 0,25 % en masse de glycérine liée et libre et seulement des métaux à l'état de trace. Ceci implique un protocole précis pour obtenir la pureté désirée.

Lorsqu'on fabrique un ester à partir d'huile ou de graisse et de monoalcool, il se forme automatiquement, selon la nature de l'huile engagée au départ, de 10 à 15 % en masse d'un produit secondaire, qui est la glycérine. Cette glycérine est vendue à un prix élevé pour des utilisations variées, mais seulement lorsqu'elle possède une grande pureté. Celle-ci est obtenue après des purifications poussées dans des unités spécialisées dans la distillation sous vide.

En résumé, la plupart des procédés commerciaux de fabrication d'esters aboutissent assez facilement à des produits bruts (esters et glycérine), qu'il faut cependant purifier de façon approfondie par divers traitements qui grèvent finalement le prix de la transformation.

Il est connu de fabriquer des esters méthyliques par les voies classiques de la catalyse homogène avec des catalyseurs solubles, comme la soude ou le méthylate de sodium, en faisant réagir une huile neutre et un alcool comme le méthanol (par exemple JAOCS 61, 343-348 (1984)). On n'arrive cependant à un produit pur utilisable comme carburant et une glycérine aux normes qu'après de très nombreuses étapes. En effet, la glycérine obtenue est polluée par les sels alcalins ou les alcoolates, si bien que l'installation de purification de la glycérine est presque aussi coûteuse que celle qui permet la fabrication de l'ester.

Les procédés par catalyse hétérogène offrent l'avantage de produire des esters et de la glycérine exempts de catalyseur donc faciles à purifier. Toutefois, il est souvent difficile d'obtenir de façon économique à la fois un ester et une glycérine de grande pureté.

Le brevet européen EP-B-0 198 243 décrit la fabrication d'ester méthylique par transestérification d'une huile avec du méthanol, en utilisant comme catalyseur une alumine ou un mélange d'alumine et d'oxyde ferreux. Toutefois, la VVH (volume d'huile injecté/volume de catalyseur/heure) est faible, la quantité de glycérine recueillie est très inférieure à celle prévue théoriquement et la pureté des esters obtenus est assez faible (comprise entre 93,5 et 98%).

Des procédés utilisant un système catalytique à base d'oxydes métalliques seuls ou associés, déposés ou non sur une alumine ont été décrits. Le brevet FR-B-2 752 242 au nom de la Demanderesse, décrit l'utilisation de catalyseurs solides et non solubles formés à partir d'oxyde de zinc et d'alumine ou d'aluminate de zinc. Les demandes de brevets EP-A- 1 505 048 et EP-A-1 593 732 également déposées au nom de la Demanderesse décrivent un procédé de transestérification d'huiles végétales ou animales au moyen de catalyseurs hétérogènes à base de mélanges d'oxydes de titane et d'alumine, d'oxyde de zirconium et d'alumine, d'oxyde d'antimoine et d'alumine ou bien de combinaison d'oxydes de zinc et de titane, d'oxyde de zinc, de titane et d'alumine, d'oxydes de bismuth, et de titane ou d'oxyde de bismuth, de titane et d'alumine.

Outre ces solides de type oxyde, un nombre grandissant de nouvelles phases basiques ont pu être utilisées pour catalyser la transestérification des huiles avec des alcools.

A titre d'exemple, De Filippis et al. (Energy & fuels 2005, 19, 225-228) suggèrent l'utilisation de phosphate de sodium pour catalyser la réaction de transestérification d'huile de colza. Bien que les auteurs indiquent que ce type de solide puisse être ré-utilisé en deux cycles successifs avec une perte d'activité de 1%, rien n'indique que la tenue des matériaux est suffisante sur de longues durées. En effet, certains problèmes dus à la présence d'eau dans la charge sont évoqués, et notamment un gonflement de ces phases et une adhérence aux parois.

Suppes et al. (Applied Catalysis A: general 257 (2004) 213-223) ont recours à divers matériaux aussi différents que des zéolithes échangées par Cs ou K ou des métaux entrant dans la composition des réacteurs, pour la transestérification de l'huile de soja.

La demande de brevet FR-2 698 101 décrit un procédé de transestérification catalytique de corps gras d'origine végétale ou animale en présence d'un catalyseur solide hétérogène insoluble tel que les phosphates de potassium et/ou de sodium à des températures comprises entre la température ambiante et la température d'ébullition de l'alcool.

Demirbas (Progress in Energy and Combustion Science 31 (2005) 466-487) présente une revue générale des procédés de production de biodiesel par des procédés de transestérification, éventuellement catalytiques, à partir d'huiles végétales.

La demande WO 2005/021697 décrit un procédé de production d'esters alkyliques d'acides gras et/ou de glycérine catalysé par des oxydes ou des mélanges d'oxydes.

Kmiya et al (Catalysis Letters Vol 94, N° 1-2, April 2004) décrit l'utilisation de phosphate de zirconium pour la réaction d'estérification d'acide acétique avec de l'éthanol.

Le brevet US-3,416,884 décrit des phosphates de zirconium cristallins.

La présente invention décrit un procédé de fabrication d'une composition d'esters alcooliques d'acides monocarboxyliques linéaires de 6 à 26 atomes de carbone et de glycérine tel que défini dans la revendication 1.

Les phosphates ou composés organophosphorés de type phosphonate ou diphosphonate de zirconium sont des solides lamellaires multifonctionnels pouvant être utilisés pour l'échange ionique, la catalyse, en tant que support catalytique ou adsorbant.

Un avantage de l'invention utilisant des catalyseurs à base de phosphates ou composés organophosphorés de type phosphonate ou diphosphonate de zirconium est leur capacité à catalyser la transestérification de corps gras avec des alcools plus lourds que le méthanol. Ainsi, on peut former des esters éthyliques, isopropyliques ou butyliques, qui présentent un intérêt car souvent les points d'écoulement des esters formés avec les alcools éthyliques, isopropyliques ou butyliques sont plus bas que ceux des esters méthyliques, le gain étant parfois de 10°C, ce qui permet d'utiliser au départ des huiles plus saturées.

Un avantage de l'invention utilisant un catalyseur à base de phosphate ou composés organophosphorés de type phosphonate ou diphosphonate de zirconium est notamment de permettre une diminution de la température de réaction, du temps de contact entre les réactifs ou du rapport alcool/corps gras par rapport à l'art antérieur, tout en améliorant le taux de conversion et en maintenant une sélectivité élevée en esters.

Un autre avantage de l'invention réside dans le fait que ces solides catalysent des réactions de transestérification et d'estérification selon un processus de catalyse hétérogène. Ainsi, le catalyseur n'est pas consommé dans la réaction et ne se retrouve pas dissous dans le milieu réactionnel. En restant sous forme solide, il est facilement séparé du milieu réactionnel sans perte de catalyseur et sans pollution du milieu réactionnel par des espèces dissoutes ou des résidus de catalyseur.

L'activité et la sélectivité de ce catalyseur n'est pas affectée par la réaction de transestérification ou d'estérification : le catalyseur est stable et recyclable dans les conditions expérimentales de la réaction. Ce type de catalyseur est compatible avec une utilisation dans un procédé industriel en continu, par exemple, en lit fixe et dans lequel la charge de catalyseur peut être utilisée pendant une très longue durée sans perte d'activité.
Le procédé de l'invention est décrit de façon plus détaillée ci-après.

### Corps gras

Les corps gras utilisés dans le procédé de l'invention correspondent à des substances naturelles ou élaborées, d'origine animale ou végétale, contenant majoritairement des triglycérides, couramment regroupés sous les termes d'huile et de graisses.

Parmi les huiles utilisables, on peut citer toutes les huiles courantes, comme les huiles de palme (concrètes ou oléines), de soja, de palmiste, de coprah, de babassu, de colza (ancien ou nouveau), de tournesol (classique ou oléique), de maïs, de coton, les huiles d'arachide, de pourghère (*Jatropha curcas),* de ricin, de lin et de crambe et toutes les huiles issues par exemple du tournesol ou du colza par modification génétique ou hybridation ou encore provenant d'algues.

On peut même utiliser des huiles de friture, d'équarrissage, des huiles animales variées, comme les huiles de poissons, de phoques, d'équarrissage, le suif, le saindoux, ou encore les graisses issues du traitement des eaux usées et même des graisses de volailles, car les esters fabriqués à partir de certains alcools comme l'alcool éthylique, isopropylique ou butylique, permettent de gagner plus de 10°C en point d'écoulement et par conséquent d'utiliser au départ des huiles plus saturées.

Parmi les huiles utilisées, on peut encore indiquer des huiles partiellement modifiées par exemple par polymérisation ou oligomérisation, comme par exemple, les "standolies" d'huile de lin, de tournesol et les huiles végétales soufflées.

Les huiles utilisées sont neutre ou acides, vierges ou recyclées.

La présence d'acide gras dans les huiles n'est pas a priori préjudiciable car les systèmes catalytiques à base de phosphate ou composé organophosphoré de type phosphonate ou diphosphonate de zirconium sont également actifs pour l'estérification et transforment également les acides gras en esters. La valeur limite en acides gras libres contenus dans les huiles se situe à un indice d'acide voisin de 10 (l'indice d'acide étant défini comme la masse en mg de KOH nécessaire au dosage de tous les acides gras libres dans 1 g d'huile). L'opérabilité du procédé dans ces conditions est proche de celle définie avec une huile à faible indice d'acide (soit inférieure à 0,2 mg de KOH/g).

Dans le cas d'huiles à très fort indice d'acide (proche de 10 mg de KOH/g), une des possibilités est de faire précéder la réaction de transestérification d'une réaction d'estérification des acides gras libres présents, soit en utilisant le même alcool que celui utilisé dans le procédé de transestérification en présence d'un acide fort comme l'acide sulfurique ou des acides sulfoniques solubles ou supportés (de type résines Amberlyst 15®), soit en utilisant de préférence de la glycérine, pour former un ester de glycérol total ou partiel, en utilisant le même catalyseur à base de phosphates ou composés organophosphorés de type phosphonates ou diphosphonates de zirconium, à pression atmosphérique et de préférence sous vide et à des températures comprises entre 150 et 220°C.

Lorsqu'on utilise des huiles de friture, qui constituent une matière première très bon marché pour produire un biodiesel, il est nécessaire d'éliminer du mélange réactionnel les polymères d'acides gras afin que le mélange d'esters réponde aux spécifications de la norme EN 14214.

### Alcool

La nature de l'alcool mis en jeu dans le procédé joue un rôle dans l'activité de transestérification.

D'une manière générale, il est possible d'utiliser divers monoalcools aliphatiques renfermant, par exemple, de 1 à 18 atomes de carbone, de préférence, de 1 à 12 atomes de carbone.

De façon encore plus préférée, le monoalcool aliphatique renferme de 1 à 5 atomes de carbone.

Le plus actif est l'alcool méthylique. Toutefois, l'alcool éthylique et les alcools isopropylique, propylique, butylique, isobutylique et même amylique, peuvent être envisagés. Des alcools plus lourds tels que l'alcool éthyl-hexylique ou l'alcool laurique peuvent également être utilisés.

On peut avantageusement ajouter aux alcools lourds de l'alcool méthylique qui facilite la réaction.

Par ailleurs, lorsqu'on prépare l'ester éthylique, on peut utiliser un mélange d'alcool éthylique et méthylique comprenant de 1 à 50% en poids, de préférence de 1 à 10% en poids, d'alcool méthylique de manière à augmenter la conversion.

### Catalyseurs:

La plupart des catalyseurs rencontrés sont sous forme de poudres, de billes, d'extrudés ou de pastilles. Ces types de mise en forme restent valables dans le cas des phases phosphates, phosphonates ou disphosphonates telles que celles que nous décrivons dans la présente invention.

Dans le cas où la technologie de réacteur impose de mettre en forme le catalyseur sous forme de billes, pastilles, granulés ou extrudés, les différents modes de mise en forme bien connus de l'homme du métier peuvent être utilisés (imprégnation, dépôts, malaxage-extrusion, granulation, pastillage...). Les exemples ci-dessous illustrent, de façon non exhaustives, certaines des méthodes envisageables.

Les poudres de phosphates ou composés organophosphorés de type phosphonate ou diphosphonate de zirconium peuvent subir une granulation avec, par exemple, utilisation de liants organiques ou inorganiques.

L'utilisation de liants, charges, agents de peptisation permet, en outre, des mises en forme sous forme d'extrudés par malaxage-extrusion.

Il est également possible de procéder à des mises en forme sphériques de zircone sans utilisation de liant via le procédé de coagulation en gouttes suivi d'une étape de modification tel que décrit dans le brevet US-6,936,175.

Les méthodes classiques de dépôt sur support préformé approprié, d'imprégnation ou de modification d'un support préformé (par exemple, tel que décrit dans le brevet US-6,936,175), bien connues de l'homme du métier, peuvent également, être avantageusement utilisées.

Un autre mode de mise en forme consiste à faire floculer le solide formé et à le sécher afin d'obtenir des granulés (US-3,056,647). Cependant, dans ce cas, de l'eau reste en partie emprisonnée dans le solide, ce qui lui confère des propriétés physiques proches de celles du silica gel.

Tous ces types de mise en forme peuvent être réalisés en présence ou absence de liant.

L'alumine, par exemple, peut être utilisée en tant que liant. Celle-ci permet d'augmenter la surface du matériau, et souvent, de créer un composé beaucoup plus stable vis-à-vis du lessivage et des contraintes mécaniques. De façon préférée, la teneur en alumine va jusqu'à 70% en poids par rapport à la masse totale du matériau mis en forme.

On peut citer parmi les sources de zirconium, les formes alcoxydes bien connues Zr(OR)₄ , R étant un groupement alkyle contenant de 3 à 18 atomes de carbone. Il est également possible d'utiliser le zirconium sous forme de sels inorganiques (ZrOCl₂, ZrOSO₄, ZrO(NO₃)₂, etc.), d'oxydes ou d'hydroxydes de zirconium.

De même, les formes colloïdales du zirconium peuvent être utilisées (par forme colloïdales, on entend que la taille des particules d'oxyde ou d'oxyhydroxyde de zirconium soit comprise entre 1 nm et 100 nm).

Enfin, les sources de zirconium peuvent être des gels issus de l'hydrolyse des sources précédentes, obtenant ainsi une forme d'oxyde de zirconium partiellement hydratée de formule chimique (ZrO₂, zH₂O) avec z compris entre 0 et 5.

Il est également avantageux d'utiliser l'oxyde de zirconium deshydraté, amorphe ou cristallisé, qui possède dans ce dernier cas des structures cristallographiques quadratique, monoclinique ou cubique.

Les sources de phosphore peuvent avoir des origines variées. De manière générale, tout type de composés organophosphorés comprenant au moins un groupe donneur de proton (P-OH par exemple) peut être employé.

De manière non limitative, on peut utiliser l'acide phosphorique H₃PO₄, ou des sels d'ammonium, de sodium, ou potassium d'acide phosphorique, des dérivés de l'acide phosphonique de formule générale ROP(OH)₂ (R étant un groupement alkyle ou aryle, fonctionnalisé ou non contenant de 1 à 20 atomes de carbone), des dérivés de l'acide diphosphonique, des pyrophosphates et des phosphinates.

La préparation de phosphates ou de composés organophosphorés de type phosphonate ou diphosphonate de métaux du groupe 4 et notamment de zirconium est connue de l'art antérieur. Elle a fait l'objet de nombreuses publications et brevets (US-6,936,175 ou US 2006/01400840). Les différentes voies de synthèses conduisant à ces solides sont applicables dans le cadre de la présente invention et les modes de préparations présentés ici ne sont, en aucun cas, restrictifs.

Ce type de catalyseur est préparé par l'une des méthodes décrites ci-après et connues de l'homme du métier.

Une méthode conventionnelle de préparation de phosphate de zirconium amorphe comprend une première étape où a lieu la réaction entre une solution aqueuse d'un sel de zirconium et de l'acide phosphorique (ou un de ses sels), ce qui conduit à la formation d'un précipité gélatineux. Après filtration, lavage et séchage, une poudre blanche ou des grains irréguliers sont obtenus qui peuvent être ensuite engagés dans une deuxième étape au cours de laquelle on réalise un échange ionique (de manière non limitative avec des sels de métaux du groupe des alcalins, alcalino-terreux, ou métaux, par exemple des sels de sodium, potassium, calcium, magnésium, baryum ou césium) afin d'obtenir un solide basique. L'échange est effectué de façon préférentielle avec des sels de métaux du groupe des alcalins ou alcalino-terreux, et plus préférentiellement avec des sels de potassium.

Dans la première étape d'une des voies de synthèse, il s'agit de synthétiser la forme acide du phosphate de zirconium Zr(O₃POH)₂.8H₂O. Celui-ci est obtenu en faisant réagir un précurseur du zirconium (ZrOCl₂.8H₂O) avec une solution d'acide phosphorique soit en portant le mélange réactionnel à reflux, soit à température ambiante sous agitation. Le solide obtenu est ensuite filtré, lavé et séché (voie 1).

Une autre voie de synthèse consiste à faire réagir dans une première étape ZrOCl₂.8H₂O avec (NH₄)₂CO₃ en formant un complexe de carbonate de zirconium. Puis, la source de phosphate (NH₄)₂HPO₄ est ajoutée et dissoute dans la solution en présence (ou non) d'un surfactant (bromure de tétradécyltriméthylammonium ou TTBr) et la solution est maintenue à 80°C pendant 3 jours pour précipitation. On procède finalement à une filtration, suivie d'un lavage d'un séchage et une calcination (voie 2).

On peut remplacer (ZrOCl₂.8H₂O) par le n-propoxyde de zirconium (Zr(OC₃H₇)₄) et le faire réagir avec l'acide phosphorique (voie 3).

Dans la seconde étape, on procède à un échange de la forme acide par des précurseurs du potassium (KCl, KOH). Après filtration et lavage, suivis d'une étape de séchage, on obtient finalement le phosphate de zirconium échangé au potassium Zr(O₃POK)₂.

Une méthode conventionnelle de préparation de phosphonate ou diphosphonate de zirconium amorphe comprend la réaction entre une solution aqueuse d'un sel de zirconium et de l'acide phosphonique ou diphosphonique (ou un de ses sels).

Ainsi, on peut faire réagir le précurseur du zirconium ZrOCl₂.8H₂O avec un acide phosphonique ou diphosphonique fonctionnalisé ou non et du NaH₂PO₄ (voie 4).

Les phosphonates et des diphosphonates sont aussi soumis à une étape d'échange ionique.

### Conditions opératoires de la réaction de transestérification

Le procédé est opéré à des températures comprises entre 130°C et 220°C, à des pressions inférieures à 100 bars avec un excès de monoalcool par rapport à la stoechiométrie corps gras/alcool.

Généralement la réaction peut être opérée selon différents modes de réalisation.

Si l'on a recours à une réaction en discontinu, on peut travailler en une ou deux étapes, c'est à dire réaliser une première réaction jusqu'à 85 % à 95 % de conversion en esters, refroidir en évaporant l'excès d'alcool, décanter la glycérine et finir la réaction en réchauffant à nouveau entre 130°C et 220°C et en ajoutant de l'alcool pour obtenir une conversion totale.

On peut aussi viser une conversion de 98 % en esters en travaillant suffisamment longtemps en une seule étape dans des conditions appropriées, par exemple en augmentant la température et/ou le rapport alcool/corps gras.

Si l'on entreprend une réaction en continu, on peut travailler avec plusieurs autoclaves et décanteurs. Dans le premier, on réalise une conversion partielle le plus souvent inférieure à 90% et généralement d'environ 85 %, puis on décante en évaporant l'alcool et en refroidissant ; dans un deuxième réacteur, on achève la réaction de transestérification dans les conditions citées en ajoutant une partie de l'alcool que l'on a évaporé précédemment. On évapore finalement dans un évaporateur l'excès d'alcool et l'on sépare la glycérine et les esters par décantation.

Ainsi, à l'issue de ces deux étapes, on obtient un biodiesel répondant aux spécifications. Le niveau de conversion est ajusté pour obtenir un ester carburant répondant aux spécifications et une glycérine de pureté élevée, en opérant en une ou deux étapes.

Si l'on choisit un procédé continu en lit fixe, on peut avec avantage travailler à des températures de 130 à 220 °C, de préférence 150 à 180 °C, à des pressions de 10 à 70 bar, la VVH étant de préférence comprise entre 0,1 et 3, de préférence de 0,3 à 2, dans la première étape et le rapport poids alcool/huile variant de 3/1 à 0,1/1.

L'introduction de l'alcool peut être avantageusement fractionnée. L'introduction à deux niveaux dans le réacteur tubulaire peut s'opérer de la façon suivante : alimentation du réacteur avec l'huile et environ les 2/3 de l'alcool à mettre en jeu, puis introduction du complément d'alcool approximativement au niveau du tiers supérieur du lit catalytique.

La tenue au lessivage est vérifiée dans la présente invention par l'absence de traces provenant du catalyseur aussi bien dans l'ester formé que dans la glycérine produite.

La recyclabilité du catalyseur est évaluée expérimentalement dans le temps.

Si l'on ne dépasse pas 220 °C, on obtient généralement un ester de même couleur que l'huile de départ et une glycérine incolore après décantation.

L'analyse des composés produits se fait, soit par chromatographie en phase gazeuse pour les esters et la glycérine, soit, plus rapidement, par chromatographie liquide par exclusion pour les esters.

L'ester et le glycérol obtenus ne contiennent pas d'impuretés issues du catalyseur. De ce fait, aucun traitement de purification ne sera appliqué pour éliminer le catalyseur ou les résidus de celui-ci contrairement aux catalyseurs fonctionnant suivant un processus homogène pour lequel le catalyseur ou ses résidus sont, après réaction, localisés dans la même phase que l'ester et/ou la glycérine.

L'ester carburant obtenu présente une teneur en monoglycérides d'au plus 0,8% en masse, en diglycérides d'au plus 0,2% en masse, en triglycérides d'au plus 0,2% en masse, en glycérine de moins de 0,25% en masse.

Par ce type de procédé, l'épuration finale est réduite au minimum, tout en permettant d'obtenir un ester aux spécifications carburant et une glycérine de pureté comprise entre 95 et 99.9% et de préférence entre 98 et 99.9%.

### EXEMPLES

Les exemples suivants illustrent l'invention sans en limiter la portée, les Exemples 5, 7 et 8 étant donnés à titre de comparaison.

Tous les exemples donnés ci-dessus ont été mis en oeuvre dans un réacteur fermé et correspondent par conséquent à une seule étape. Pour obtenir un biodiesel répondant aux spécifications, il serait nécessaire de procéder à l'issue de cette première étape à une décantation en évaporant l'alcool et en refroidissant, puis d'achever la réaction de transestérification en ajoutant la partie de l'alcool évaporé.

L'huile utilisée dans ces exemples est de l'huile de colza dont la composition en acides gras est la suivante :

**Tableau 1 : Composition de l'huile de colza.**

| Glycéride d'acides gras | Nature de la chaîne grasse | % en masse |
|---|---|---|
| Palmitique | C16 :0 | 5 |
| Palmitoléique | C16 :1 | < 0,5 |
| Stéarique | C18 :0 | 2 |
| Oléique | C18 :1 | 59 |
| Linoléique | C18 :2 | 21 |
| Linoléique | C18 :3 | 9 |
| Arachidique | C20 :0 | < 0,5 |
| Gadoléique | C20 :1 | 1 |
| Béhénique | C22 :0 | < 0,5 |
| Erucique | C22 :1 | < 1 |

Cependant, toute autre huile d'origine végétale ou animale pourrait donner des résultats analogues.
Le catalyseur utilisé dans les Exemples 1, 3, 5, 6 et 7 a été préparé en faisant réagir dans un premier temps un précurseur du zirconium (ZrOCl₂.8H₂O) avec une solution d'acide phosphorique (voie 1) puis en procédant à l'étape d'échange ionique décrite précédemment afin d'obtenir la phase basique.

### Exemple 1 : Transestérification d'huiles végétâtes (huile de colza) par le méthanol à partir d'un catalyseur solide de type phosphate de zirconium Zr(OPO₃K)₂ à 180°C.

On introduit dans un réacteur fermé à température ambiante 25 g d'huile de colza, 25 g de méthanol et 1 g de catalyseur sous forme de poudre. Le ratio massique méthanol/ huile est donc de 1, ce qui correspond à un ratio molaire de 27,5. Le réacteur est ensuite fermé, agité (200 trs/min) et chauffé à 180°C à l'aide d'un agitateur magnétique chauffant. La température du milieu réactionnel est stabilisée à 180°C après 20 minutes de chauffe. La pression est la pression autogène de l'alcool à la température de travail. Le suivi de la réaction est commencé lorsque la température du milieu réactionnel a atteint la consigne. Des prélèvements sont effectués de manière régulière afin de suivre l'avancement de la réaction. Après 6 h de réaction, l'agitation est arrêtée et le réacteur laissé à refroidir jusqu'à température ambiante. Les prélèvements effectués ainsi que l'effluent final sont lavés par une solution aqueuse saturée en NaCl, puis après décantation, la phase organique supérieure est analysée par chromatographie par perméation de gel (GPC). Le tableau suivant résume les résultats obtenus.

| | | **Prélèvements (en h)** | | |
|---|---|---|---|---|
| | | **0^{b}** | **0,4** | **0,6** |
| **% massique dans la phase organique^{a}** | Triglycérides | 26 | 5 | 1 |
| | Diglycérides^{c} | 17 | 7 | 3 |
| | Monoglycéride | 13 | 10 | 5 |
| | Esters méthyliques d'huile végétale | 44 | 79 | 90 |

| | | | | |
|---|---|---|---|---|
| ^{a} déterminé par GPC ^{b} t=0 lorsque le milieu réactionnel est à température ^{c} % représentant les diglycérides et stérols | | | | |

La conversion des triglycérides commence alors que le milieu réactionnel n'a pas atteint 180°C (44% d'esters à t0). L'équilibre thermodynamique est atteint très rapidement (environ 40 min) après que le milieu réactionnel soit à 180°C. La conversion (estimée par rapport aux triglycérides, conversion=1-m_{finale} (triglycérides)/mᵢₙᵢₜᵢₐₗₑ (triglycérides)) est de 99% à 40 min.

Le lessivage du catalyseur dans la phase ester est négligeable (la teneur en phophore et potassium, estimée par la technique plasma à couplage inductif (IPC) est inférieure à 5 ppm). Ce résultat est valable pour tous les exemples suivants en ce qui concerne les phosphates de zirconium.

### Exemple 2 : Transestérification d'huiles végétales (huile de colza) par le méthanol à partir d'un catalyseur solide de type phosphate de zirconium Zr(OPO₃K)₂ à 170°C.

On répète l'Exemple 1 en utilisant 25 g d'huile de colza, 25 g de méthanol et 1 g de catalyseur sous forme de poudre. La réaction est conduite à 170°C, la température du milieu réactionnel étant stabilisée à 170°C après 20 minutes de chauffe. Le tableau suivant résume les résultats obtenus.

| | | **Prélèvements (en h)** | | | |
|---|---|---|---|---|---|
| | | **0^{b}** | **0,4** | **0,6** | **1** |
| **% massique dans la phase organique^{a}** | Triglycérides | 48 | 15 | 3 | 1 |
| | Diglycérides^{c} | 17 | 11 | 5 | 3 |
| | Monoglycéride | 9 | 12 | 8 | 5 |
| | Esters méthyliques d'huile végétale | 26 | 62 | 83 | 90 |

| | | | | | |
|---|---|---|---|---|---|
| ^{a} déterminé par GPC ^{b} t=0 lorsque le milieu réactionnel est à température ^{c} % représentant les diglycérides et stérols | | | | | |

La conversion des triglycérides commence alors que le milieu réactionnel n'a pas atteint 170°C (26% d'esters à t0). L'équilibre thermodynamique est atteint très rapidement (en 60 min environ). La conversion (estimée par rapport aux triglycérides) est de 99% à 60 min.

### Exemple 3 : Transestérification d'huiles végétales (huile de colza) par le méthanol à partir d'un catalyseur solide de type phosphate de zirconium Zr(OPO₃K)₂ à 160°C.

On répète l'exemple 1 en utilisant 25 g d'huile de colza, 25 g de méthanol et 1 g de catalyseur sous forme de poudre. La réaction est conduite à 160°C, la température du milieu réactionnel étant stabilisée à 160°C après 20 minutes de chauffe. Le tableau suivant résume les résultats obtenus.

| | | **Prélèvements (en h)** | | | |
|---|---|---|---|---|---|
| | | **0^{b}** | **0,4** | **0,6** | **1,5** |
| **% massique dans la phase organique^{a}** | Triglycérides | 47 | 24 | 9 | 1 |
| | Diglycérides^{c} | 18 | 15 | 9 | 3 |
| | Monoglycéride | 7 | 12 | 11 | 5 |
| | Esters méthyliques d'huile végétale | 28 | 49 | 71 | 90 |

| | | | | | |
|---|---|---|---|---|---|
| ^{a} déterminé par GPC ^{b} t=0 lorsque le milieu réactionnel est à température ^{c} % représentant les diglycérides et stérols | | | | | |

L'équilibre thermodynamique est atteint très rapidement (en 1,5 h environ). La conversion (estimée par rapport aux triglycérides) est de 99% en 1,5 h.

### Exemple 4 : Transestérification d'huiles végétales (huile de colza) par le méthanol à partir d'un catalyseur solide de type phosphate de zirconium Zr(OPO₃K)₂ à 150°C.

On répète l'Exemple 1 en utilisant 25 g d'huile de colza, 25 g de méthanol et 1 g de catalyseur sous forme de poudre. La réaction est conduite à 150°C, la température du milieu réactionnel étant stabilisée à 150°C après 15 minutes de chauffe. Le tableau suivant résume les résultats obtenus.

| | | **Prélèvements (en h)** | | | |
|---|---|---|---|---|---|
| | | **0^{b}** | **0,4** | **0,6** | **2,1** |
| **% massique dans la phase organique^{a}** | Triglycérides | 69 | 36 | 19 | 1 |
| | Diglycérides^{c} | 15 | 18 | 14 | 3 |
| | Monoglycéride | 4 | 11 | 13 | 5 |
| | Esters méthyliques d'huile végétale | 12 | 35 | 54 | 90 |

| | | | | | |
|---|---|---|---|---|---|
| ^{a} déterminé par GPC ^{b} t=0 lorsque le milieu réactionnel est à température ^{c} % représentant les diglycérides et stérols | | | | | |

Les 99 % de conversion sont atteints en 2,1 h à 150°C.

### Exemple 5 (comparatif): Transestérification de l'huile de colza par le méthanol en présence d'aluminate de zinc (ZnAl₂O₄) sous forme de poudre à 150°C.

On répète l'Exemple 1 en utilisant 25 g d'huile de colza, 25 g de méthanol et 1 g de catalyseur sous forme de poudre. La réaction est conduite à 150°C, la température du milieu réactionnel étant stabilisée à 150°C après 15 minutes de chauffe. Le tableau suivant résume les résultats obtenus.

| | | **Prélèvements (en h)** | | |
|---|---|---|---|---|
| | | **0^{b}** | **4** | **6** |
| **% massique dans la phase organique^{a}** | Triglycérides | 94 | 69 | 61 |
| | Diglycérides^{c} | 4 | 17 | 19 |
| | Monoglycéride | 0 | 2 | 3 |
| | Esters méthyliques d'huile végétale | 2 | 12 | 16 |

| | | | | |
|---|---|---|---|---|
| ^{a} déterminé par GPC ^{b} t=0 lorsque le milieu réactionnel est à température ^{c} % représentant les diglycérides et stérols | | | | |

Cette exemple montre clairement que l'aluminate de zinc catalyse la réaction de transestérification beaucoup plus lentement qu'un phosphate de zirconium, puisque les 99 % de conversion ne seront atteints qu'au bout de 60 h.

### Exemple 6 : Transestérification d'huiles végétales (huile de colza) par le méthanol à partir d'un catalyseur solide de type phosphate de zirconium Zr(OPO₃K)₂ à 140°C.

On répète l'Exemple 1 en utilisant 25 g d'huile de colza, 25 g de méthanol et 3 g de catalyseur sous forme de poudre. La réaction est consuite à 140°C, la température du milieu réactionnel étant stabilisée à 140°C après 30 minutes de chauffe. Le tableau suivant résume les résultats obtenus.

| | | **Prélèvements (en h)** | | |
|---|---|---|---|---|
| | | **0^{b}** | **4** | **6** |
| **% massique dans la phase organique^{a}** | Triglycérides | 19 | 0 | 0 |
| | Diglycérides^{c} | 8 | 2 | 2 |
| | Monoglycéride | 8 | 4 | 4 |
| | Esters méthyliques d'huile végétale | 64 | 94 | 94 |

| | | | | |
|---|---|---|---|---|
| ^{a} déterminé par GPC ^{b} t=0 lorsque le milieu réactionnel est à température ^{c} % représentant les diglycérides et stérols | | | | |

Cet exemple montre que le fait d'augmenter la masse de catalyseur permet de travailler à des températures plus faibles en ayant une conversion totale très rapidement (moins de 4 h).

### Exemple 7 (non conforme à l'invention) : Transestérification d'huiles végétales (huile de colza) par le méthanol à partir d'un catalyseur solide de type phosphate de zirconium acide Zr(OPO₃H)₂ à 200°C.

On répète l'Exemple 1 en utilisant 25 g d'huile de colza, 25 g de méthanol et 3 g de catalyseur sous forme de poudre. La réaction est conduite à 200°C, la température du milieu réactionnel étant stabilisée à 200°C après 40 minutes de chauffe. Le tableau suivant résume les résultats obtenus.

| | | **Prélèvements (en h)** | | |
|---|---|---|---|---|
| | | **0^{b}** | **4** | **6** |
| **% massique dans la phase organique^{a}** | Triglycérides | 93 | 45 | 27 |
| | Diglycérides^{c} | 5 | 25 | 26 |
| | Monoglycéride | 0 | 7 | 12 |
| | Esters méthyliques d'huile végétale | 2 | 23 | 35 |

| | | | | |
|---|---|---|---|---|
| ^{a} déterminé par GPC ^{b} t=0 lorsque le milieu réactionnel est à température ^{c} % représentant les diglycérides et stérols | | | | |

L'activité de la forme acide est très faible, car seulement 35% d'esters sont obtenus après 6h de réaction, à 200°C et avec 3 g de solide. Cette quantité d'esters correspond approximativement à celle obtenue en l'absence de catalyseur (catalyse purement thermique). La forme acide du phosphate de zirconium est donc très peu active

### Exemple 8 (comparatif) Transestérification d'huiles végétales (huile de colza) par le méthanol à partir d'oxyde de magnésium MgO à 150°C.

On répète l'exemple 5 en utilisant 25 g d'huile de colza, 25 g de méthanol et 1 g de catalyseur sous forme de poudre. La réaction est conduite à 150°C , la température du milieu réactionnel étant stabilisée à 150°C après 20 minutes de chauffe. Le tableau suivant résume les résultats obtenus.

| | | **Prélèvements (en h)** | | |
|---|---|---|---|---|
| | | **0^{b}** | **4** | **6** |
| **% massique dans la phase organique^{a}** | Triglycérides | 83 | 5 | 1 |
| | Diglycérides^{c} | 10 | 6 | 3 |
| | Monoglycéride | 1 | 8 | 5 |
| | Esters méthyliques d'huile végétale | 5 | 80 | 91 |

| | | | | |
|---|---|---|---|---|
| ^{a} déterminé par GPC ^{b} t=0 lorsque le milieu réactionnel est à température ^{c} % représentant les diglycérides et stérols | | | | |

Cette exemple montre clairement que l'oxyde de magnésium catalyse la réaction de transestérification beaucoup plus lentement qu'un phosphate de zirconium, puisque les 99 % de conversion ne sont atteints qu'au bout de 6 h.

### Exemple 9: Influence du ratio molaire méthanol/huile R=27,5

Les exemples précédents ont été réalisés avec un ratio méthanol/huile massique de 1, ce qui correspond en réalité à un ratio molaire de 27,5. Les exemples suivants illustrent l'influence de ce ratio sur l'activité catalytique d'un catalyseur de type phosphate de zirconium et soulignent les bonnes performances du catalyseur même à de faibles ratios.

On répète l'Exemple 1 en utilisant 25 g d'huile de colza, 25 g de méthanol et 1 g de catalyseur sous forme de poudre. La réaction est conduite à 160°C, la température du milieu réactionnel étant stabilisée à 160°C après 22 minutes de chauffe. Le tableau suivant résume les résultats obtenus.

| | | **Prélèvements (en h)** | | | |
|---|---|---|---|---|---|
| | | **0^{b}** | **0,4** | **0,6** | **2** |
| **% massique dans la phase organique^{a}** | Triglycérides | 39 | 11 | 2 | 0,1 |
| | Diglycérides^{c} | 19 | 9 | 4 | 2 |
| | Monoqlycéride | 10 | 12 | 7 | 4 |
| | Esters méthyliques d'huile végétale | 31 | 68 | 87 | 94 |

| | | | | | |
|---|---|---|---|---|---|
| ^{a} déterminé par GPC ^{b} t=0 lorsque le milieu réactionnel est à température ^{c} % représentant les diglycérides et stérols | | | | | |

Avec un ratio molaire de R=27,5, la conversion est de 98% en 40 min.

### Exemple 10: Influence du ratio molaire méthanol/huile R=20

On répète l'Exemple 9 en utilisant 28,9 g d'huile de colza, 21,1 g de méthanol et 1 g de catalyseur sous forme de poudre. La réaction est conduite à 160°C, la température du milieu réactionnel étant stabilisée à 160°C après 22 minutes de chauffe. Le tableau suivant résume les résultats obtenus.

| | | **Prélèvements (en h)** | | | |
|---|---|---|---|---|---|
| | | **0^{b}** | **0,4** | **0,6** | **2** |
| **% massique dans la phase organique^{a}** | Triglycérides | 52 | 24 | 10 | 0,3 |
| | Diglycérides^{c} | 20 | 17 | 10 | 2 |
| | Monoglycéride | 6 | 13 | 13 | 6 |
| | Esters méthyliques d'huile végétale | 21 | 46 | 66 | 92 |

| | | | | | |
|---|---|---|---|---|---|
| ^{a} déterminé par GPC ^{b} t=0 lorsque le milieu réactionnel est à température ^{c} % représentant les diglycérides et stérols | | | | | |

Avec R=20, la conversion est de 90% en 40 min, et est quasi totale en moins de deux heures.

### Exemple 11: Influence du ratio molaire méthanol/huile R=14

On répète l'Exemple 9 en utilisant 33,1 g d'huile de colza, 16,8 g de méthanol et 1 g de catalyseur sous forme de poudre. La réaction est conduite à 160°C, la température du milieu réactionnel étant stabilisée à 160°C après 22 minutes de chauffe. Le tableau suivant résume les résultats obtenus.

| | | **Prélèvements (en h)** | | | |
|---|---|---|---|---|---|
| | | **0^{b}** | **0,4** | **0,6** | **2** |
| **% massique dans la phase organique^{a}** | Triglycérides | 60 | 33 | 20 | 2 |
| | Diglycérides^{c} | 18 | 19 | 15 | 4 |
| | Monoglycéride | 5 | 12 | 14 | 10 |
| | Esters méthyliques d'huile végétale | 17 | 36 | 50 | 84 |

| | | | | | |
|---|---|---|---|---|---|
| ^{a} déterminé par GPC ^{b} t=0 lorsque le milieu réactionnel est à température ^{c} % représentant les diglycérides et stérols | | | | | |

Avec R=14, la conversion est de 80% en 40 min, et reste quasi totale (98%) en deux heures.

### Exemple 12: Influence du ratio molaire méthanol/huile R=10

On répète l'Exemple 9 en utilisant 36,7 g d'huile de colza, 13,3 g de méthanol et 1 g de catalyseur sous forme de poudre. La réaction est conduite à 160°C, la température du milieu réactionnel étant stabilisée à 160°C après 22 minutes de chauffe. Le tableau suivant résume les résultats obtenus.

| | | **Prélèvements (en h)** | | | |
|---|---|---|---|---|---|
| | | **0^{b}** | **0,4** | **0,6** | **2** |
| **% massique dans la phase organique^{a}** | Triglycérides | 69 | 45 | 30 | 6 |
| | Diglycérides^{c} | 16 | 20 | 19 | 9 |
| | Monoglycéride | 3 | 9 | 13 | 15 |
| | Esters méthyliques d'huile végétale | 12 | 25 | 38 | 70 |

| | | | | | |
|---|---|---|---|---|---|
| ^{a} déterminé par GPC ^{b} t=0 lorsque le milieu réactionnel est à température ^{c} % représentant les diglycérides et stérols | | | | | |

Avec un ratio molaire méthanol/huile de 10, la conversion est de 70% en 40 min, et est de 96% en deux heures. Ce faible ratio ne permet donc pas d'atteindre une conversion totale en deux heures. Néanmoins, le système catalytique reste plus rapide qu'avec un catalyseur à base de MgO ou un aluminate de zinc utilisant de large excès de méthanol (R=27,5).

### Exemple 13: Influence de la nature de l'alcool

On répète l'Exemple 1 en utilisant 25 g d'huile de colza, 25 g d'éthanol et 3 g de catalyseur de type phosphate de zirconium sous forme de poudre. La réaction est conduite à 200°C, la température du milieu réactionnel étant stabilisée à 200°C après 60 minutes de chauffe. Le tableau suivant résume les résultats obtenus.

| | | **Prélèvements (en h)** | | |
|---|---|---|---|---|
| | | **0^{b}** | **4** | **6** |
| **% massique dans la phase organique^{a}** | Triglycérides | 59 | 1 | 0,1 |
| | Diglycérides^{c} | 20 | 4 | 3 |
| | Monoglycéride | 5 | 13 | 9 |
| | Esters méthyliques d'huile végétale | 16 | 81 | 88 |

| | | | | |
|---|---|---|---|---|
| ^{a} déterminé par GPC ^{b} t=0 lorsque le milieu réactionnel est à température ^{c} % représentant les diglycérides et stérols | | | | |

Après 6 h de réaction, la conversion est totale, et le rendement en esters éthyliques est de 88%.

### Exemple 14: Influence de la nature de l'alcool

On répète l'Exemple 13 en utilisant 25 g d'huile de colza, 25 g d' éthanol et 3 g de catalyseur de type phosphate de zirconium sous forme de poudre. La réaction est conduite à 180°C, la température du milieu réactionnel étant stabilisée à 180°C après 30 minutes de chauffe. Le tableau suivant résume les résultats obtenus.

| | | **Prélèvements (en h)** | | |
|---|---|---|---|---|
| | | **0^{b}** | **4** | **6** |
| **% massique dans la phase organique^{a}** | Triglycérides | 83 | 13 | 4 |
| | Diglycérides^{c} | 11 | 14 | 7 |
| | Monoglycéride | 1 | 17 | 17 |
| | Esters méthyliques d'huile végétale | 5 | 56 | 72 |

| | | | | |
|---|---|---|---|---|
| ^{a} déterminé par GPC ^{b} t=0 lorsque le milieu réactionnel est à température ^{c} % représentant les diglycérides et stérols | | | | |

Après 6 h de réaction, la conversion est de 96% à 180°C, et le rendement en esters éthyliques est de 72%.

## Revendications

1. Procédé de fabrication d'une composition d'esters alcooliques d'acides monocarboxyliques linéaires de 6 à 26 atomes de carbone et de glycérine, dans lequel on fait réagir un corps gras d'origine végétale ou animale avec un monoalcool aliphatique renfermant de 1 à 18 atomes de carbone, à une température comprise entre 130° et 220°C, à une pression inférieure à 100 bars et avec un excès de monoalcool par rapport à la stoechiométrie corps gras/alcool, en présence d'au moins un catalyseur hétérogène basique choisi parmi le phosphate, le phosphonate ou diphosphonate de zirconium, préparé par réaction entre une solution d'un sel de zirconium et d'un acide choisi respectivement parmi l'acide phosphorique, l'acide phosphonique ou l'acide diphosphonique, le produit obtenu étant soumis à une étape d'échange ionique.

2. Procédé selon la revendication 1 dans lequel ledit monoalcool aliphatique renferme de 1 à 12 atomes de carbone.

3. Procédé selon la revendication 1 dans lequel ledit monoalcool aliphatique renferme de 1 à 5 atomes de carbone.

4. Procédé selon l'une des revendications 1 à 3 dans lequel l'alcool mis en jeu est un mélange d'alcool éthylique et méthylique, comprenant de 1 à 50% en poids, de préférence de 1 à 10% en poids d'alcool méthylique.

5. Procédé selon l'une des revendications 1 à 4 dans lequel l'huile de départ est choisie parmi les huiles de palme (concrètes ou oléines), de soja, de palmiste, de coprah, de babassu, de colza ancien ou nouveau, de tournesol classique ou oléique, de maïs, de coton, les huiles d'arachide, de pourghère, de ricin, de lin et de crambe, d'algues et les huiles du tournesol ou du colza obtenus par modification génétique ou par hybridation, les huiles partiellement modifiées par polymérisation ou oligomérisation.

6. Procédé selon l'une des revendications 1 à 4 dans lequel l'huile de départ est choisie parmi les huiles de friture, d'équarrissage, les huiles de poissons, de phoques, les graisses de volailles, le suif, le saindoux, les graisses issues du traitement des eaux usées.

7. Procédé selon l'une des revendications 1 à 6 **caractérisé en ce que** le catalyseur est sous forme de poudre, d'extrudés, de billes ou de pastilles.

8. Procédé selon l'une des revendications 1 à 7 dans lequel on utilise de l'alumine comme liant, dans des proportions allant jusqu'à 70% en poids de la masse totale du matériau mis en forme..

9. Procédé selon l'une des revendications 1 à 8 dans lequel les sources de zirconium sont choisies parmi les formes alcoxydes Zr(OR)₄, R étant un groupement alkyle contenant de 3 à 18 atomes de carbone, les sels inorganiques, les oxydes ou hydroxydes de zirconium.

10. Procédé selon la revendication 9 dans lequel les sources de zirconium se présentent sous forme d'oxyde partiellement hydratée de formule ZrO₂, zH₂O avec z compris entre 0 et 5.

11. Procédé selon la revendication 9 dans lequel la source de zirconium est de l'oxyde de zirconium déshydraté, amorphe ou cristallisé.

12. Procédé selon l'une des revendications 1 à 11 dans lequel la source de phosphore est un composé organophosphoré comprenant au moins un groupe donneur de proton.

13. Procédé selon la revendication 12 dans lequel les sources de phosphore sont choisies parmi l'acide phosphorique, les sels d'acide phosphorique, des dérivés de l'acide phosphonique de formule générale ROP(OH)₂, R étant un groupement alkyle, aryle fonctionnalisé ou non contenant de 1 à 20 atomes de carbone, des dérivés de l'acide diphosphonique, des pyrophosphates et phosphinates.

14. Procédé selon l'une des revendications 1 à 13 dans lequel le catalyseur est Zr(OPO₃K)₂ et est obtenu en faisant réagir du ZrOCl₂,8H₂O avec une solution d'acide phosphorique soit en portant le mélange réactionnel à reflux, soit à température ambiante sous agitation, puis on procède à l'étape d'échange ionique.

15. Procédé selon l'une des revendications 1 à 14 dans lequel la réaction est mise en oeuvre en discontinu.

16. Procédé selon l'une des revendications 1 à 15 dans lequel la réaction est mise en oeuvre en continu, en lit fixe ou avec des autoclaves et décanteurs en série.

17. Procédé selon la revendication 16 dans lequel la réaction est mise en oeuvre en lit fixe, à une pression comprise entre 10 et 70 bars et à une VVH comprise entre 0,1 et 3, avec un rapport poids alcool/corps gras compris entre 3/1 et 0,1/1.

18. Procédé selon la revendication 16 dans lequel on opère une réaction en continu, avec plusieurs autoclaves et décanteurs, et où on réalise, dans le premier, une conversion partielle inférieure à 90%, puis on décante en évaporant l'alcool et en refroidissant ; dans un deuxième réacteur, on achève la réaction de transestérification dans les conditions décrites à la revendication 1 en ajoutant une partie de l'alcool que l'on a évaporé précédemment puis on évapore dans un évaporateur l'excès d'alcool et l'on sépare la glycérine et les esters par décantation.

## Patentansprüche

1. Verfahren zur Herstellung einer Zusammensetzung aus Alkoholestern linearer Monocarbonsäuren mit 6 bis 26 Kohlenstoffatomen und Glycerin, wobei ein Fett pflanzlichen oder tierischen Ursprungs mit einem aliphatischen Monoalkohol umgesetzt wird, der 1 bis 18 Kohlenstoffatome enthält, bei einer Temperatur im Bereich zwischen 130 ° und 220 °C, bei einem Druck von weniger als 100 Bar und mit einem Monoalkoholüberschuss, bezogen auf die Fett/Alkohol-Stöchiometrie, in Gegenwart mindestens eines heterogenen basischen Katalysators, ausgewählt aus Phosphat, Zirconiumphosphonat oder Zirconiumdiphosphonat, das durch Umsetzung einer Lösung eines Zirconiumsalzes und einer Säure hergestellt wurde, die jeweils aus Phosphorsäure, Phosphonsäure oder Diphosphonsäure ausgewählt ist, wobei das erhaltene Produkt einem Schritt zum Ionenaustausch unterzogen wird.

2. Verfahren nach Anspruch 1, wobei der aliphatische Monoalkohol 1 bis 12 Kohlenstoffatome enthält.

3. Verfahren nach Anspruch 1, wobei der aliphatische Monoalkohol 1 bis 5 Kohlenstoffatome enthält.

4. Verfahren nach einem der Ansprüche 1 bis 3, wobei der eingesetzte Alkohol ein Gemisch aus Ethyl- und Methylalkohol ist, das 1 bis 50 Gew.-%, bevorzugt 1 bis 10 Gew.-% Methylalkohol umfasst.

5. Verfahren nach einem der Ansprüche 1 bis 4, wobei das Ausgangsöl ausgewählt ist aus (konkreten oder oleinhaltigen) Palmölen, Sojaölen, Palmkernölen, Kokosölen, Babassuölen, alten und neuen Rapsölen, herkömmlichen oder ölsäurehaltigen Sonnenblumenölen, Maisölen, Baumwollsamenölen, Erdnussölen, Purgiernussölen, Rizinusölen, Leinölen und Krambeölen, Algenölen und Sonnenblumenölen oder Rapsölen, die durch gentechnische Veränderung oder durch Hybridisierung erhalten wurden, durch Polymerisation oder Oligomerisation teilweise modifizierten Ölen.

6. Verfahren nach einem der Ansprüche 1 bis 4, wobei das Ausgangsöl ausgewählt ist aus Frittierölen, Kadaverölen, Fischölen, Robbenölen, Geflügelfetten, Talg, Schweineschmalz, Fetten, die aus der Behandlung von Abwässern stammen.

7. Verfahren nach einem der Ansprüche 1 bis 6, **dadurch gekennzeichnet, dass** der Katalysator in Form von Pulver, Extrudaten, Kugeln oder Pellets vorliegt.

8. Verfahren nach einem der Ansprüche 1 bis 7, wobei Aluminiumoxid, in Anteilen, die bis zu 70 Gew.-% der Gesamtmasse des geformten Materials erreichen, als Bindemittel verwendet wird.

9. Verfahren nach einem der Ansprüche 1 bis 8, wobei die Zirconiumquellen ausgewählt sind aus Alkoxidformen Zr(OR)₄, wobei R eine Alkylgruppe ist, die 3 bis 18 Kohlenstoffatome enthält, anorganischen Salzen, Zirconiumoxiden oder - hydroxiden.

10. Verfahren nach Anspruch 9, wobei die Zirconiumquellen eine teilweise hydratisierte Oxidform mit der Formel ZrO₂, zH₂O aufweisen, wobei z im Bereich zwischen 0 und 5 liegt.

11. Verfahren nach Anspruch 9, wobei die Zirconiumquelle amorphes oder kristallisiertes, dehydratisiertes Zirconiumoxid ist.

12. Verfahren nach einem der Ansprüche 1 bis 11, wobei die Phosphorquelle eine phosphororganische Verbindung ist, die mindestens eine Protonendonorgruppe umfasst.

13. Verfahren nach Anspruch 12, wobei die Phosphorquellen ausgewählt sind aus Phosphorsäure, Phosphorsäuresalzen, Phosphonsäurederivaten mit der allgemeinen Formel ROP(OH)₂, wobei R eine funktionalisierte oder nicht funktionalisierte Alkylgruppe oder Arylgruppe ist, die 1 bis 20 Kohlenstoffatome enthält, Diphosphonsäurederivate, Pyrophosphate und Phosphinate.

14. Verfahren nach einem der Ansprüche 1 bis 13, wobei der Katalysator Zr(OPO₃K)₂ ist und erhalten wird, indem ZrOCl₂,8H₂O mit einer Phosphorsäurelösung umgesetzt wird, entweder indem das Reaktionsgemisch rückflussgekocht oder bei Umgebungstemperatur gerührt wird, dann erfolgt der Schritt zum Ionenaustausch.

15. Verfahren nach einem der Ansprüche 1 bis 14, wobei die Reaktion diskontinuierlich durchgeführt wird.

16. Verfahren nach einem der Ansprüche 1 bis 15, wobei die Reaktion kontinuierlich im Festbett oder mit in Reihe geschalteten Autoklaven und Dekantern durchgeführt wird.

17. Verfahren nach Anspruch 16, wobei die Reaktion im Festbett bei einem Druck im Bereich zwischen 10 und 70 Bar und mit einer HSV im Bereich zwischen 0,1 und 3 mit einem Gewichtsverhältnis Alkohol/Fettstoff im Bereich zwischen 3/1 und 0,1/1 durchgeführt wird.

18. Verfahren nach Anspruch 16, wobei eine kontinuierliche Reaktion mit mehreren Autoklaven und Dekantern durchgeführt wird, und wobei im ersten eine teilweise Umwandlung von weniger als 90 % ausgeführt wird, dann dekantiert wird, indem der Alkohol verdampft und abgekühlt wird; in einem zweiten Reaktor die Veresterungsreaktion unter den in Anspruch 1 beschriebenen Bedingungen beendet wird, indem ein Teil des Alkohols zugegeben wird, der zuvor verdampft wurde, dann in einem Verdampfer der Alkoholüberschuss verdampft wird und das Glycerin und die Ester durch Dekantieren getrennt werden.

## Claims

1. A method of manufacturing a composition of alcohol esters of linear monocarboxylic acids with 6 to 26 carbon atoms and glycerin, wherein a fatty substance of vegetable or animal origin is reacted with an aliphatic monoalcohol having 1 to 18 carbon atoms, wherein the temperature ranges between 130°C and 220°C, the pressure is below 100 bars, and excess monoalcohol is used in relation to the fatty substance/alcohol stoichiometry, in the presence of at least one basic heterogeneous catalyst based on zirconium phosphate, zirconium phosphonate or zirconium diphosphonate, prepared by reaction between a solution of a zirconium salt and an acid chosen among respectively phosphoric, phosphonic or diphosphonic acid, the product obtained being subjected to an ion exchange stage.

2. A method as claimed in claim 1, wherein said aliphatic monoalcohol comprises 1 to 12 carbon atoms.

3. A method as claimed in claim 1, wherein said aliphatic monoalcohol comprises 1 to 5 carbon atoms.

4. A method as claimed in any one of claims 1 to 3, wherein the alcohol used is a mixture of ethyl and methyl alcohol, comprising 1 to 50 wt.%, preferably 1 to 10 wt.% methyl alcohol.

5. A method as claimed in any one of claims 1 to 4, wherein the initial oil is selected from among palm oil (concrete or olein), soybean oil, palm nut oil, copra oil, babassu oil, rapeseed oil, old or new, sunflower oil, conventional or oleic, corn oil, cotton oil, peanut oil, pourgher oil, castor oil, linseed oil and crambe oil, oils obtained from algae and the sunflower and rapeseed oils obtained by genetic engineering or hybridization, oils partly modified by polymerization or oligomerization.

6. A method as claimed in any one of claims 1 to 4, wherein the initial oil is selected from among waste kitchen oil, slaughterhouse oil, fish oil, seal oil, fowl fat, tallow, lard, fat from sewage treatment.

7. A method as claimed in any one of claims 1 to 6, **characterized in that** the catalyst comes in form of powder, extrudates, balls or pellets.

8. A method as claimed in any one of claims 1 to 7, wherein alumina is used as the binder, in proportions up to 70 wt.% of the total mass of the material formed.

9. A method as claimed in any one of claims 1 to 8, wherein the zirconium sources are selected from among the alkoxide forms Zr(OR)₄, R being an alkyl group containing 3 to 18 carbon atoms, inorganic salts, zirconium oxides or hydroxides.

10. A method as claimed in claim 9, wherein the zirconium sources come in form of a partly hydrated oxide of formula ZrO₂, zH₂O, z ranging between 0 and 5.

11. A method as claimed in claim 9, wherein the zirconium source is dehydrated zirconium oxide, amorphous or crystallized.

12. A method as claimed in any one of claims 1 to 11, wherein the phosphorus source is an organophosphorous compound comprising at least one proton donor group.

13. A method as claimed in claim 12, wherein the phosphorus sources are selected from among phosphoric acid, phosphoric acid salts, phosphonic acid derivatives of general formula ROP(OH)₂, R being an alkyl or aryl group, functionalized or not, having 1 to 20 carbon atoms, diphosphonic acid derivatives, pyrophosphates and phosphinates.

14. A method as claimed in any one of claims 1 to 13, wherein the catalyst is Zr(O₃POK)₂ and it is obtained by reacting ZrOCl₂.8H₂O with a phosphoric acid solution either by refluxing the reaction mixture or at ambient temperature under stirring, then the ion exchange stage is carried out.

15. A method as claimed in any one of claims 1 to 14, wherein the reaction is carried out on a discontinuous basis.

16. A method as claimed in any one of claims 1 to 15, wherein the reaction is carried out on a continuous basis, in a fixed bed or with autoclaves and decanters in series.

17. A method as claimed in claim 16, wherein the reaction is carried out in a fixed bed, at a pressure ranging between 10 and 70 bars and at an LHSV ranging between 0.1 and 3, with an alcohol/fatty substance weight ratio ranging between 3/1 and 0.1/1.

18. A method as claimed in claim 16, wherein the reaction is carried out on a continuous basis using several autoclaves and decanters, and wherein a partial conversion below 90 % is performed in a first reactor, then decanting is achieved by evaporating the alcohol and by cooling, wherein the transesterification reaction is completed in a second reactor under the conditions mentioned in claim 1 by adding part of the alcohol previously evaporated, then evaporating the excess alcohol in an evaporator and then separating the glycerin and the esters by decantation.
